# EUROPEAN PATENT SPECIFICATION

(11) **EP 4 106 861 B1**
(45) Date of publication and mention of the grant of the patent: **11.12.2024**
(21) Application number: 21702465.2
(22) Date of filing: 28.01.2021
(51) Int. Cl.: A61N 1/37, A61N 1/36, A61N 1/39, A61N 1/08

(54) **IMPLANTABLE MEDICAL DEVICE CONFIGURED FOR DETECTING A PRESENCE OF AN MRI DEVICE**
IMPLANTIERBARE MEDIZINISCHE VORRICHTUNG MIT KONFIGURATION ZUR ERKENNUNG DES VORHANDENSEINS EINER MRT-VORRICHTUNG
DISPOSITIF MÉDICAL IMPLANTABLE CONÇU POUR DÉTECTER LA PRÉSENCE D'UN DISPOSITIF IRM

(30) Priority: 21.02.2020 EP 20158713
(43) Date of publication of application: 28.12.2022
(73) Proprietor: BIOTRONIK SE & Co. KG, 12359 Berlin (DE)
(72) Inventor: KAMENZ, Uwe, 13507 Berlin (DE)
(74) Representative: Biotronik Corporate Services SE
(86) International application number: PCT/EP2021/051974
(87) International publication number: WO 2021/165008

(56) References cited:
- US-A1- 2009 157 146
- US-A1- 2012 053 652
- US-A1- 2015 352 354

## Description

The invention relates to an implantable medical device according to the preamble of claim 1 and to a method for operating an implantable medical device.

An implantable medical device shall be configured for implantation into a patient. An implantable medical device in this context may for example be a pacemaker device for providing for a pacing action in a patient's heart, or a defibrillator device, such as an implantable cardioverter-defibrillator (ICD), for providing for a defibrillation, or a sensor device having a sensing function for monitoring for example a cardiac activity of a patient. In addition, the implantable medical device may be a recording device, such as a loop recorder, or a transmitter device for transmitting signals to a device external to a patient, or a data carrier, such as a patient identification device.

An implantable medical device of this kind comprises a sensing device for sensing a magnetic field, and a processing device configured to detect the presence of an MRI device based on measurement values obtained from the sensing device.

An implantable medical device, for example a stimulation device, such as a pacemaker device or a defibrillator device, generally is configured to output electrical stimulation energy for performing a therapeutic stimulation action. An implantable medical device in addition may be configured to sense electrical signals relating e.g. to cardiac activity, in particular to trigger and clock a stimulation action. If an implantable medical device is introduced into a magnetic field of an MRI device, it can be assumed that electrical signals are induced on leads and other conducting parts of the implantable medical device, such electrical signals causing a disturbance of the operation of the medical device. For example for an implantable medical device in the shape of a pacemaker device or a defibrillator device, a disturbance of a stimulation action may cause an erroneous stimulation and hence may have a significant impact on a therapy of a patient, which needs to be avoided.

There hence is a need to detect whether a patient carrying an implantable medical device is in the vicinity of an MRI device, such that, if this is the case, operation of the medical device may be modified in order to reduce a risk for a malfunction of the implantable medical device.

US 9,364,663 B2 discloses an implantable medical device including a power supply, a sensing device and/or a stimulation device. An MR detection unit may identify an MR-typical journey of an implantable medical device on a patient bed of an MRI device, based on a change over time of measurement values of at least two magnetic field sensors and a difference between the measured values of the at least two magnetic field sensors. Herein, if measurement values of the at least two magnetic field sensors exceed a threshold, a presence of an MRI device is assumed.

US 9,981,124 B2 discloses an implantable medical device having a first magnetic field direction sensor located at a first location within a housing and configured to generate a signal representative of a first direction of a magnetic field at the first location, and a second magnetic field direction sensor located at a second location within the housing and configured to generate a signal representative of a second direction of the magnetic field at the second location. Based on signals obtained via the first magnetic field direction sensor and the second magnetic field direction sensor it is concluded for a presence of an MRI device.

Generally, an implantable medical device is configured to interact electromagnetically with external devices, such as a programming device or a triggering device which are placed outside of a patient and can be brought into vicinity of an implantable medical device. By electromagnetic interaction of an external device with the implantable medical device a communication between the implantable medical device and the external device can be established, for example for programming the implantable medical device or for transferring data from the implantable medical device to the external device.

An implantable medical device shall be configured to be able to distinguish between an external device, which shall interact with the implantable medical device, and another magnetic device, in particular an MRI device, which may cause a disturbance of an operation of the implantable medical device and hence needs to be detected in order to be able to suitably modify an operation of the implantable medical device in order to reduce a risk for a malfunction of the implantable medical device.

In document US 2012/0053652 A1 an implantable medical device is described including a lead, a monitoring module, a multi-function conductive (MFC) coil, and a field detection module. The monitoring module identifies cardiac events based on the cardiac signals and directs stimulus pulses to be delivered to the heart through one or more electrodes connected to the lead. The MFC coil has an electric characteristic that varies based on exposure of the coil to an external magnetic field. The field detection module detects exposure of the coil to the external magnetic field by applying a field detection signal to the coil and identifying a change in the electric characteristic of the MFC coil. The field detection module switches operation of the monitoring module to an MR safe mode based on the change that is identified.

In document US 2009/0157146 A1 an operating mode of an implantable medical device is described that can be selected using at least one of a current or a voltage provided in response to a magnetic field sensed using a Hall effect sensor.

It is an object of the instant invention to provide an implantable medical device and a method for operating an implantable medical device which in an easy-to-implement way allow for a reliable detection of a presence of an MRI device.

This object is achieved by means of an implantable medical device comprising the features of claim 1.

Accordingly, the processing device of the implantable medical device is configured to conclude that an MRI device is present if a multiplicity of measurement values indicates an increase of a strength of the magnetic field.

An MRI device uses a strong, constant magnet field having for example a nominal magnetic field strength of above 1T, for example 1.5T, 3T or 7T. By superimposing the constant magnetic field with time-varying magnetic gradient fields a magnetic resonant effect is induced, which may be detected using RF detection coils for picking up signals within a patient's body to conduct an imaging of the patient.

Herein, when a patient shall undergo an MRI examination, the patient typically is placed on a patient bed of the MRI device and, by moving the patient bed into a bore of the MRI device, is placed with a body part to be examined inside of the bore of the MRI device.

If a patient carrying an implantable medical device, such as a cardiac stimulation device, shall undergo an MRI examination, the implantable medical device shall be enabled to detect that the patient is approaching an MRI device such that operation of the implantable medical device may suitably be modified in order to reduce a risk of an impact of the MRI device, in particular the strong magnetic field of the MRI device, on the operation of the implantable medical device. If it is detected that a patient is in the range of the magnetic field of an MRI device, for example a sensing function of the implantable medical device for triggering or clocking a stimulation action can be switched off, such that the implantable medical device for example may be operated in a mode using no sensing of electrical activity in a region of interest, for example in a patient's heart, such that a therapy, for example a pacing action, without a clocking based on external sensing signals is carried out (for example in a so-called VV0 mode of a pacemaker device). In addition or alternatively, a therapy function may be switched off, for example a shock function of a defibrillator device, such that an (erroneous) shock during an MRI examination is avoided.

For detecting whether a patient carrying an implantable medical device is in the vicinity of an MRI device, measurement values of a sensing device for detecting a magnetic field are examined. Herein, if it is found that a multiplicity of measurement values obtained from the sensing device of the implantable medical device indicate an increase of the strength of the magnetic field, it is assumed that a patient is placed on a patient bed of an MRI device and is moved, by moving the patient's bed with a substantially constant velocity, into the bore of the MRI device.

If a patient is placed on a patient bed of an MRI device and the patient bed is moved into the bore of the MRI device, the magnetic field strength at the location of the implantable medical device will steadily increase as the patient is moved into the MRI device. This steady increase of the magnetic field strength at the location of the implantable medical device may be observed by monitoring measurement values of the sensing device. If multiple temporally offset measurement values indicate an increase of the magnetic field strength, it may be concluded that the patient is moved into the MRI device, such that operation of the implantable medical device may suitably be modified.

Hence, by observing measurement values obtained from the sensing device and by determining whether an increase of the magnetic field strength indicative of a steady movement of the patient into an MRI device is present, a situation in which a patient is moved into an MRI device may be distinguished from a situation in which an external device other than an MRI device, such as a programming device or the like, is brought into vicinity of the implantable medical device in order to interact with the implantable medical device. If a patient shall undergo an MRI examination, operation of the implantable medical device shall be monitored in order to avoid a malfunction. If instead an external device is brought into the vicinity of the implantable medical device, potentially a communication with the external medical device shall be established, or a particular function of the implantable medical device shall be triggered, the external device for example providing a wake-up signal to the implantable medical device.

In one embodiment, the sensing device is configured to provide measurement values at a predefined sampling rate. For example, using the sensing device measurements may be conducted at a specified frequency, for example at a frequency in between 1 Hz and 50 Hz, for example 4 Hz, such that the sensing device outputs (discrete) measurement values at the predefined sampling rate. The measurement values are input to the processing device, which analyzes the measurement values and, if multiple measurement values indicate a (steady) increase of the magnetic field strength it is concluded for the presence of an MRI device.

The sensing device may output measurement values indicative of the magnetic field strength. The measurement values herein may be output as digits, each digit corresponding to a specified voltage value, for example 1 mV. As the sensing device may have a nonlinear characteristic curve, the voltage value may nonlinearly (dependent on the magnetic field strength) indicate a certain change in the magnetic field strength.

In one embodiment, the processing device is configured to conclude that an MRI device is present if a multiplicity of consecutive measurement values indicate an increase of the strength of the magnetic field. Hence, if it is found that for consecutive measurement values the magnetic field increases in strength, it is concluded that the patient is moved into an MRI device and hence an MRI device is identified.

For example, it may be concluded for the presence of an MRI device if for a multiplicity of consecutive measurement values an increase of the strength of the magnetic field exceeding, for each measurement value, a predefined margin is detected. Hence, if for a number of consecutive measurement values the magnetic field strength increases for each measurement value by at least a certain margin, it is concluded for the presence of an MRI device, as it is found that the strength of the magnetic field steadily increases due for example to a movement of a patient bed into the MRI device. Alternatively or in combination, the presence of an MRI device can be concluded if the magnetic field increases over a certain number of consecutive measurements without considerable drops.

In one embodiment, the processing device is configured to increment a counter value if a measurement value indicates an increase of the strength of the magnetic field. Herein, for example, the processing device may be configured to increment the counter value if a measurement value indicates an increase of the strength of the magnetic field exceeding a predefined count threshold. Hence, each time a measurement value obtained from the sensing device indicates that the strength of the magnetic field has changed by a certain minimum margin corresponding to the count threshold, the counter value is incremented, such that the counter value counts the times that the measurement values indicate a substantial increase of the magnetic field strength.

Herein, the counter value may be incremented by one from an initial start value, for example 0, if the measurement value indicates an increase of the magnetic field strength exceeding a first margin. Subsequently, if subsequent measurement values indicate an increase of the magnetic field strength exceeding a second margin corresponding to the count threshold, for each increment the counter value is again increased by 1 such that the counter value indicates the number of measurement occasions at which the count threshold has been exceeded. The first margin may be larger than the second margin. If measurement values are output in digits, the first margin may for example correspond to a value between 2 and 5 digits, for example 3 digits, whereas the second margin corresponding to the count threshold may correspond to a value between 1 and 3 digits, for example 2 digits.

The counter value hence is derived from the measurement values. The counter value in each case is incremented by one (if applicable) for a measurement value obtained from the sensing device, independent of the actual increase amount of the magnetic field strength.

According to an embodiment of the present invention, the processing device is configured to decrement the counter value if a measurement value indicates a decrease of the strength of the magnetic field, wherein the decrease of the magnetic field strength exceeds a predefined countdown threshold. For instance, if measurement values are output in digits, the countdown threshold may correspond to a drop of 2 or 3 digits.

According to an embodiment of the present invention, the counter value may not fall below zero.

The counter may be decremented in one step by one or more count values, e.g. two, three etc. By choosing a higher decrement count value, the counter is decremented faster. If the processing device is configured to perform certain actions in association with counter decrementation, e.g. withholding detection of the presence of an MRI machine or switching the implantable device from an MRI-mode back to normal operation, setting a higher decrement value (i.e. after a small number of counter decrements) will cause that actions to be triggered faster than if a smaller decrement value was chosen.

In one embodiment, the processing device is configured to conclude that an MRI device is present if the counter value is incremented for a predefined number of times (larger than 1, preferably larger than 2), for example a predefined number of consecutive times. The predefined number of times, may for example have a value in between 2 and 20, for example 5. If it is found that the counter value is steadily incremented for the predefined number of (consecutive) times, it is concluded for the presence of an MRI device, as it is found that the magnetic field strength at the location of the implantable medical device steadily increases.

In addition or alternatively, the processing device may be configured to conclude that an MRI device is present if the counter value reaches or exceeds a predefined detection threshold. Hence, even if the counter value is not found to steadily increase over a predefined number of (consecutive) times, it may be concluded for the presence of an MRI device if the counter value reaches or exceeds the detection threshold. Hence, if the counter value becomes as large or larger than the detection threshold, it in any case is assumed that an MRI device is present.

In one embodiment, the processing device is configured to reset the counter value to a start value, for example 0, if the counter value is not incremented in a predefined period of time. The predefined period of time may for example range from 1 to 20 minutes, for example 10 minutes. If it hence is found that the counter value no longer increases, which is observed over a suitable time span, the counter value is reset, because it is assumed that the implantable medical device is not or no longer in the range of an MRI device.

Alternatively or in addition, the processing device may be configured to reset the counter value to a start value, for example 0, if a measurement value indicates a decrease of the strength of the magnetic field by a value larger than a predefined reset threshold. If it is found that the magnetic field strength substantially decreases, it can be assumed that the implantable medical device is not or no longer in the range of an MRI device. Hence, the counter value is reset such that a detection procedure is started anew to detect the presence of an MRI device in case of an increase of the magnetic field strength.

In an embodiment of the present invention, the processing device is configured to reset the counter value to a start value in case a predefined number of consecutive measurement values each indicate a decrease of the strength of the magnetic field. The counter value is reset if each of the measurement values show a decrease by a value which is larger than a predefined countdown threshold.

Setting predefined number of consecutive measurement values for deciding on counter reset has the advantage that an exit of an MRI environment of an MRI machine can be detected with a high reliability. By exiting the MRI environment, the magnetic field strength of the patient (and the implantable medical device) normally decreases in a controlled speed, which should be detected by the processing device. A single measurement of a decrease of the magnetic field followed by an increase, or single decreases alternating with increases, would in some cases not be sufficient to indicate an exit from the MRI environment. For instance, such events could be caused by a disturbance of the magnetic field measurements, or by an adjustment of the patient table of the MRI by the physician or nurse. For example, the number of consecutive measurement values can be set to 2 to 10 measurements. Particularly reliable results could be produced when the number was set to 3 consecutive measurements. The predefined countdown threshold may correspond to a drop of 2 or 3 digits.

In one embodiment, the sensing device may be a GMR sensor (GMR stands for giant magnetoresistance). The sensing device hence is configured to measure a magnetic field strength and output a voltage value indicative of a magnetic field strength present at the location of the sensing device.

In one embodiment, the processing device is configured to modify operation of the implantable medical device if it is concluded that an MRI device is present. The modification of operation may in particular include a switching-off of a sensing function of the implantable medical device, or a switching-off or modification of a therapy function. For example, for a stimulation device such as a pacemaker device a pacing function may be modified such that a pacing without an external trigger or clocking is achieved, for example corresponding to a so-called VV0 mode of a pacemaker device. For a defibrillator device, for example a shock function may be switched off such that a shock function is not available while a patient carrying the device undergoes an MRI examination.

According to an embodiment of the present invention, the processing device is configured to terminate said modifying of operation of the implantable medical device if the counter value is reset. A reset of the counter value is associated with exit of the MRI environment, so that modification of the operation of the implantable device becomes unnecessary, the implantable device may return to a normal non-MRI mode.

According to an embodiment, the processing device is configured to start a termination timer if counter value is reset. The modifying of operation of the implantable medical device is terminated as the termination timer reaches a predefined time margin. Applying a termination timer is advantageous for situations in which the counter value is reset before the patient exits the MRI environment entirely. In such cases, the modification of the operation of the implantable medical device is maintained after counter value reset for a certain time span as safety measure for the patient. The time span is defined by said time margin and can be set to e.g. 30, 60, 90, 120 seconds. In particular, setting the time margin to 60 seconds produced reliable results. If during that time span the counter value increases again, so that presence of an MRI device is redetected, the modifying of the operation of the implantable device may be maintained (i.e. not terminated after expiry of the time span). If no presence of an MRI device is redetected during said time span or until the termination timer reached the time margin, the modifying of the operation of the implantable device is terminated and the implantable medical device returns to a normal, non-MRI operational mode. Back to the normal operational mode, the implantable medical device resumes to apply the routines for detecting the presence of an MRI machine according to the present invention. The termination timer is set to its start value and will be retriggered next time the counter value is reset.

Alternatively, according to an embodiment of the present invention, the processing device is configured to conclude that an MRI device is present if a at least a part of a curve associated with the multiplicity of consecutive measurement values corresponds to at least a part of a reference curve. In the context of the present invention, a part of a curve corresponds to a part of a second curve if their shapes are similar by a certain degree. For example, a curve generated by the multiplicity of consecutive measurement values obtained by the sensing device can be compared to a reference curve by calculating the difference of gradients of a part of both curves, and comparing the difference to a threshold. In case the difference stays below the threshold, the two parts of the curves may correspond to each other. If the difference exceeds the threshold, there may be no correspondence between the sections of both curves. Alternatively, the parts of both curves can be compared by calculating the difference between the areas under the curves. If the difference stays below a certain threshold, the two parts of the curves may have a certain degree of similarity and they correspond to each other. The reference curve may be stored in the implantable medical device in the manufacturing process, and is accessible to the processing device. For instance, the reference curve may be generated via numerous measurements of the magnetic field strength of an object which enters an MRI machine on a patient table, generating numerous test curves. The test curves are used to compute an average curve, which is stored as reference curve. As an alternative or in combination, the reference curve may be adapted or updated continuously by newly generated curves associated with the multiplicity of consecutive measurement values from the sensing device.

The object is also achieved by a method for operating an implantable medical device, the method comprising: sensing a magnetic field using a sensing device of the implantable medical device, and detecting a presence of an MRI device, using a processing device of the implantable medical device, based on measurement values obtained from the sensing device. Herein, it is concluded, by the processing device, that an MRI device is present if a multiplicity of measurement values indicates an increase of a strength of the magnetic field.

The advantages and advantageous embodiments of the implantable medical device as described above equally apply also to the method, such that it shall be referred to the above.

Various features and advantages of the present invention may be more readily understood with reference to the following detailed description and the embodiments shown in the drawings. Herein,
- Fig. 1: shows a schematic illustration of an implantable medical device in a patient;
- Fig. 2: shows a schematic drawing of an MRI device;
- Fig. 3: shows a schematic drawing of an implantable medical device;
- Fig. 4: shows graphs of measurement values output by a sensing device of the implantable medical device, and a counter value derived from the measurement values; and
- Fig. 5: shows characteristic curves of a sensing device in the shape of a GMR sensor.

Subsequently, embodiments of the invention shall be described in detail with reference to the drawings. In the drawings, like reference numerals shall designate functionally similar structural elements, if appropriate.

It is to be noted that the embodiments are not limiting for the invention, but merely represent illustrative examples.

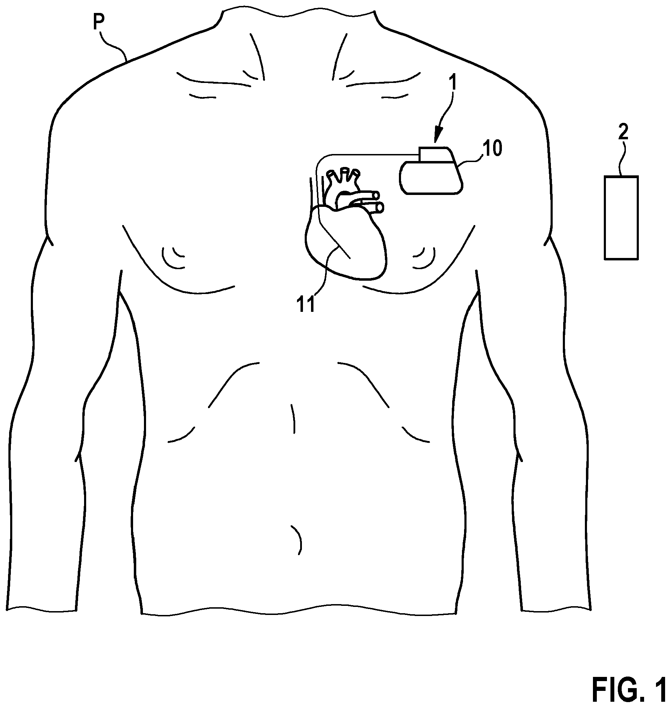

Fig. 1 shows a schematic illustration of an implantable medical device 1, for example in the shape of a stimulation device, such as a pacing device or a defibrillation device. The implantable medical device 1 may for example comprise a generator 10 which for example, as illustrated in Fig. 1, may be subcutaneously implanted into a patient, wherein an electrode 11 is connected to the generator 10 and extends from the generator 10 towards a region of interest, for example the patient's heart, such that a therapy function may be provided at the region of interest, for example in the patient's heart.

An implantable medical device 1 of this kind may for example be configured to provide a therapy function over a prolonged period of time, for example a pacing function or a defibrillator function. The implantable medical device 1, for this, may be permanently implanted into a patient P and may function in a substantially autarkic manner, wherein a communication connection may be established with the implantable medical device 1 using an external device 2, for example to program the implantable medical device 1 or to transfer, using for example telemetry, data from the implantable medical device 1 to the external device 2.

An external device 2 may communicate with the implantable medical device 1 using electromagnetic means, for example by establishing an inductive coupling in between the implantable medical device 1 and the external device 2.

The external device 2 may alternatively be a permanent magnet which may be brought into the vicinity of the implantable medical device 1 in order to trigger an action of the implantable medical device 1, for example for waking up the medical device 1.

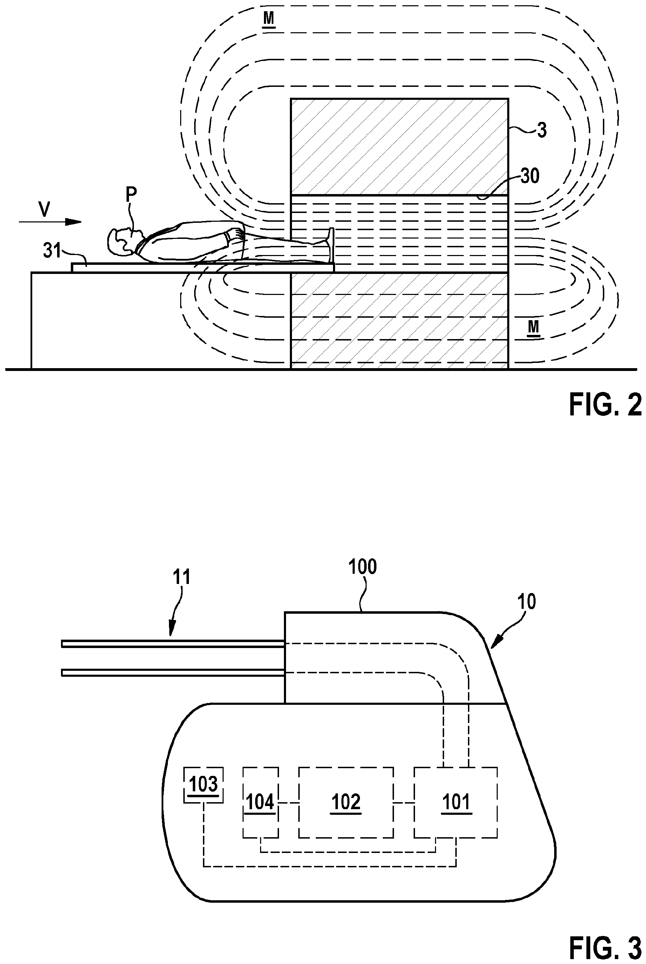

If, as schematically illustrated in Fig. 2, a patient P shall undergo an MRI examination using an MRI device 3, the patient P, carrying an implantable medical device 1, is introduced into a bore 30 of the MRI device 3 by placing the patient P on a patient bed 31 and by continuously moving, using an electro-motoric drive of the patient bed 31, the patient P into the bore 30 in a movement direction V. When moving the patient P into the bore 30 of the MRI device 3, the patient P herein is brought into the range of a constant magnetic field M of the MRI device 3, the magnetic field M generally having a maximum magnetic field strength (corresponding to the magnetic flux density B and indicated usually in Tesla [T]) within the bore 30. Hence, when the patient P is moved into the bore 30 of the MRI device 3, the magnetic field strength of the magnetic field M at the location of the implantable medical device 1 will steadily increase.

Caused by a magnetic field M of an MRI device 3, electrical signals may be induced within an implantable medical device 1. Hence, it shall be detected if an implantable medical device 1 comes into the range of an MRI device 3, such that operation of the implantable medical device 1 can suitably be modified in order to avoid a disturbance of operation by the MRI device 3.

Fig. 3 illustrates an embodiment of a generator 10 of an implantable medical device 1 for example in the shape of a stimulation device such as a pacemaker device or a defibrillator device. Included in a housing of the generator 10 is a processing device 101, implemented e.g. by electronic circuitry on a circuit board, which serves to control operation of the generator 10 for transmission of electrical stimulation energy via the electrodes 11 connected to a connector block 100 of the generator 10, and for analyzing sense signals received e.g. via the electrodes 11 to provide for a therapy aligned to an activity of for example the patient's heart.

The generator 10 further comprises an energy storage 102 in the shape of a battery, a sensing device 103 for example in the shape of a GMR sensor for sensing magnetic fields, and a communication device 104 for establishing a communication connection for example to an external device 2, as illustrated in Fig. 1.

The sensing device 103 is connected to the processing device 101 and is configured to conduct measurements yielding measurement values indicative of a magnetic field strength at the location of the sensing device 103. The sensing device 103 may for example be configured to conduct measurements at a specified sampling rate, for example at a rate in between 1 Hz and 50 Hz, for example 4 Hz. The sensing device 103 provides (discrete) measurement values to the processing device 101, which are analyzed by the processing device 101 and are used for controlling operation of the implantable medical device 1.

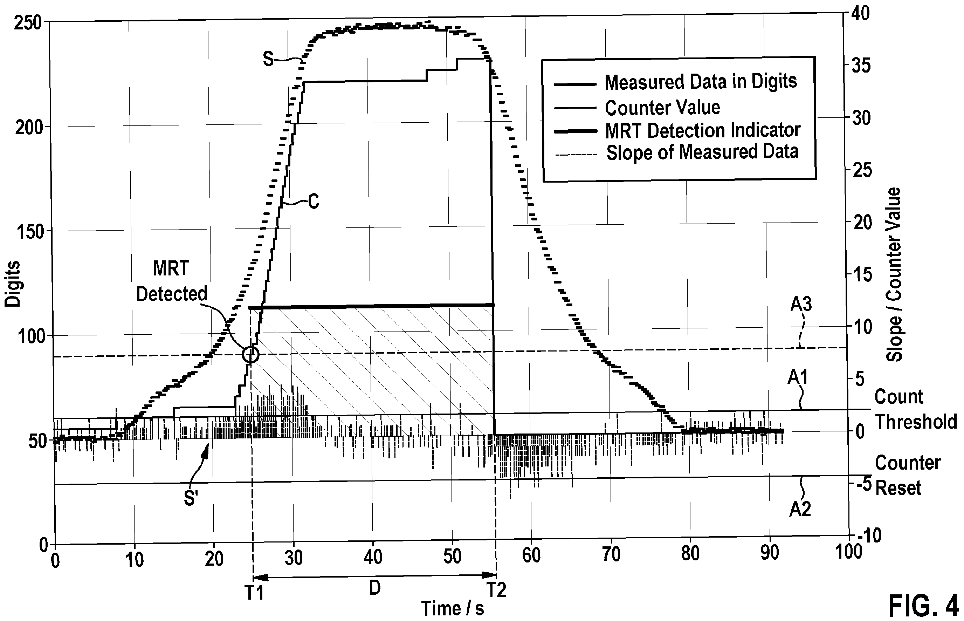

Referring now to Fig. 4, when a patient P is moved into the bore 30 of an MRI device 3 by electromotively moving the patient's bed 31 in a movement direction V, the sensing device 103 outputs measurement values S over time. The measurement values S herein are provided at the specified sampling rate in digits, one digit corresponding to a voltage value of 1 mV as output by the sensing device 103 (left vertical axis in Fig. 4).

Fig. 4 in addition to the measurement values S shows corresponding change values S' (corresponding to the discrete slope of the measurement values S) over time, the change values S' indicating a change between an actual measurement value and the previous measurement value (right vertical axis in Fig. 4).

Based on the measurement values S and the change values S' a counter value C is derived. Herein, the counter value C initially is set to 0. If a measurement value S is obtained which corresponds to a change value S' exceeding a specified initial margin, for example 3 digits, the counter value is incremented from 0 to 1, wherein subsequently the counter value C is incremented by one each time the change value S' associated with the actual measurement value S exceeds a predefined count threshold A1, corresponding for example to a value of 2 digits.

If it is found that a counter value C reaches, at time T1, a detection threshold A3, it is assumed that an MRI device 3 is present and hence a flag "MRI detected" may be set by the processing device 101. Accordingly, in a phase D following time T1 operation of the implantable medical device 1 may be modified in order to account for the presence of the magnetic field M of the MRI device 3 and to suitably adjust operation of the implantable medical device 1 in order to reduce a risk for disturbances caused by the magnetic field M of the MRI device 3.

Alternatively or in addition to comparing the counter value C to a detection threshold A3, the processing device 101 may be configured to determine whether the counter value C for a predefined number of consecutive times is incremented, indicating that the change value S' for consecutive measurement values S repeatedly exceeds the count threshold A1. The predefined number of consecutive times may for example be set to 5. If, hence, the counter value C is consecutively incremented by 1 for five consecutive measurements, it is assumed that presumably the magnetic field steadily increases as the patient P is moved into an MRI device 3 and, hence, an MRI device 3 is present.

In the example of Fig. 4, at time T1 an MRI device 3 is assumed to be detected, and hence in phase D following time T1 a suitable indicator flag is set. The counter value C, due to the further increase of the magnetic field M and the corresponding rise of the measurement values S, continues to be incremented, until a plateau is reached, corresponding for example to a rest period in which the patient P rests in the bore 30 of the MRI device 3 and the patient bed 31 is at a standstill.

If the patient P is moved out of the bore 30, the magnetic field strength at the location of the implantable medical device 1 will decrease, which is detected and indicated by the measurement values S. If it is found that a change value S' corresponding to a measurement value S indicates a decrease of the magnetic field strength by a margin exceeding a reset threshold A2, as it is the case at time T2 in Fig. 4, the counter value C is reset to 0. In addition, the "MRI detected" indicator flag is canceled, such that the phase D indicating a detected MRI device 3 lasts between time T1 and time T2, as illustrated in Fig. 4.

Alternatively or in addition, if it is found that the counter value C is not further incremented over a predefined period of time, for example ranging from 1 minute to 20 minutes, the counter value C may also be reset to 0.

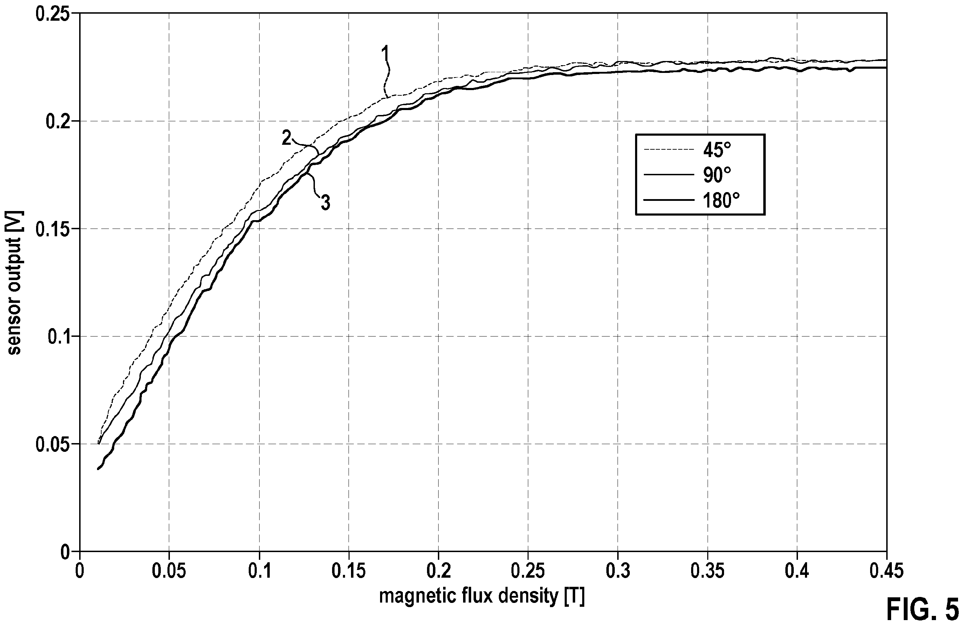

Referring now to Fig. 5, the sensing device 103, for example implemented using two GMR sensors rotated by 90° to one another, has characteristic curves O1, 02, O3 which are nonlinear and are substantially independent of an angular orientation of the sensing device 103 (illustrated by the fact that the characteristic curves O1, 02, O3 corresponding to different angular orientations of the sensing device 103 are substantially identical). The sensing device 103 outputs digits, each digit corresponding to a voltage value of 1 mV. Herein, the digits are nonlinearly correlated to the magnetic field strength (magnetic flux density, measured in Tesla), as this is indicated by the characteristic curves O1, O2, O3.

If the sensing device 103 detects the presence of an MRI device 3 and correspondingly sets the "MRI detected" flag, the processing device 101 is configured to modify operation of the implantable medical device 1. For example, if the implantable medical device 1 is a stimulation device, for example a pacemaker device, a therapy function can be modified, for example by switching off a sensing function in order to provide a pacing action without a clocking by cardiac activity. If the implantable medical device 1 is for example a defibrillator device, a shock function may be switched off, in particular in order to avoid an erroneous shock while a patient P is within an MRI device 3, due to signals induced within the implantable medical device 1 by the magnetic field M of the MRI device 3.

An implantable medical device as concerned herein may in particular be a cardiac stimulation device such as a pacemaker device or a defibrillator device, but may also be a stimulation device for example for a neuro-stimulation. In addition, the implantable medical device may for example be a sensor device, such as an implantable pressure sensor or the like.

Because a magnetic field strength, for determining whether an MRI device is present, is analyzed over time by observing multiple (preferably consecutive) measurement values, a detection reliability for detecting an MRI device - in differentiation to another external device - may be improved. In this way the number of wrong detections of an MRI device may be substantially reduced, hence improving therapy for a patient and the patient's well-being.

A functionality as described herein may be implemented by a change in software of an implantable medical device, such that implementation may be easy and cost-effective without requiring a change in hardware.

### List of Reference Numerals

- 1: Implantable medical device (pacemaker device)
- 10: Generator
- 100: Connector block
- 101: Processing device
- 102: Energy storage
- 103: Sensing device
- 104: Communication device
- 11: Electrode
- 2: External device
- 3: MRI device
- 30: Bore
- 31: Patient bed
- A1: Count threshold
- A2: Reset threshold
- A3: Detection threshold
- C: Counter value
- D: Phase
- M: Magnetic field (magnetic flux density)
- O1, O2, O3: Characteristic curve (sensor output)
- P: Patient
- S: Measurement values (in digits)
- S': Slope
- V: Moving direction

## Claims

1. An implantable medical device (1), comprising: a sensing device (103) for sensing a magnetic field (M); and a processing device (101) configured to detect a presence of an MRI device (3) based on measurement values (S) obtained from the sensing device (103); wherein the processing device (101) is configured to conclude that an MRI device (3) is present if a multiplicity of measurement values (S) obtained from the sensing device (103) indicates an increase of a strength of the magnetic field (M), **characterized in that** the sensing device (103) is configured to conduct measurements at a specified sampling rate in between 1 Hz and 50 Hz, in particular at 4 Hz, and to provide measurement values (S) at the specified sampling rate,
wherein the processing device (101) is configured to increment a counter value (C) if a measurement value (S) indicates an increase of the strength of the magnetic field (M) and to reset the counter value (C) to a start value if the counter value (C) is not increased in a predefined period of time.

2. The implantable medical device (1) of claim 1, wherein
the processing device (101) is configured to conclude that an MRI device (3) is present if a multiplicity of consecutive measurement values (S) indicate an increase of the strength of the magnetic field (M).

3. The implantable medical device (1) of one of the preceding claims, wherein
the processing device (101) is configured to increment the counter value (C) if a measurement value (S) indicates an increase of the strength of the magnetic field (M) exceeding a predefined count threshold (A1).

4. The implantable medical device (1) of one of the preceding claims, wherein
the processing device (101) is configured to decrement the counter value (C) if a measurement value (S) indicates a decrease of the strength of the magnetic field (M), wherein the decrease of the magnetic field strength exceeds a predefined countdown threshold.

5. The implantable medical device (1) of one of the preceding claims, wherein
the processing device (101) is configured to conclude that an MRI device (3) is present if the counter value (C) is increased for a predefined number of times or if the counter value (C) reaches or exceeds a predefined detection threshold (A3).

6. The implantable medical device (1) of claim 5, wherein
the processing device (101) is configured to conclude that an MRI device (3) is present if the counter value (C) is increased for a predefined number of consecutive times or if the counter value (C) reaches or exceeds a predefined detection threshold (A3).

7. The implantable medical device (1) of one of the preceding claims, wherein
that the processing device (101) is configured to reset the counter value (C) to a start value if a measurement value (S) indicates a decrease of the strength of the magnetic field (M) by a value larger than a predefined reset threshold (A2).

8. The implantable medical device (1) of one of the preceding claims , wherein
the processing device (101) is configured to reset the counter value (C) to a start value if a predefined number of consecutive measurement values (S) each indicate a decrease of the strength of the magnetic field (M) by a value larger than a predefined countdown threshold.

9. The implantable medical device (1) of one of the preceding claims, wherein
the processing device (101) is configured to modify operation of the implantable medical device (1) if it is concluded that an MRI device (3) is present.

10. The implantable medical device (1) of claim 9 referring to one of the claims 4 to 7, wherein the processing device (101) is configured to
- terminate modification of operation of the implantable medical device (1) if counter value (C) is reset, or
- start a termination timer if counter value (C) is reset, and terminate modification of operation of the implantable medical device (1) when the termination timer reached a predefined time margin.

11. The implantable medical device (1) of one of the preceding claims, wherein
the processing device (101) is configured to conclude that an MRI device (3) is present if a at least a part of a curve associated with the multiplicity of consecutive measurement values (S) corresponds to at least a part of a reference curve.

12. Method for operating an implantable medical device (1), comprising: sensing a magnetic field (M) using a sensing device (103) of the implantable medical device (1); and detecting a presence of an MRI device (3), using a processing device (101) of the implantable medical device (1), based on measurement values (S) obtained from the sensing device (103); concluding, by the processing device (101), that an MRI device (3) is present if a multiplicity of measurement values (S) obtained from the sensing device (103) indicates an increase of a strength of the magnetic field (M), **characterized by** conducting measurements at a specified sampling rate in between 1 Hz and 50 Hz, in particular at 4 Hz, and providing measurement values (S) at the specified sampling rate,
incrementing, by the processing device (101), a counter value (C) if a measurement value (S) indicates an increase of the strength of the magnetic field (M) and resetting, by the processing device (101), the counter value (C) to a start value if the counter value (C) is not increased in a predefined period of time.

## Patentansprüche

1. Implantierbare medizinische Vorrichtung (1), umfassend: eine Erfassungsvorrichtung (103) zum Erfassen eines Magnetfelds (M); und eine Verarbeitungsvorrichtung (101), die dazu ausgestaltet ist, ein Vorhandensein einer MRI-Vorrichtung (3) auf der Grundlage von Messwerten (S) zu erkennen, die von der Erfassungsvorrichtung (103) erhalten werden; wobei die Verarbeitungsvorrichtung (101) dazu ausgestaltet ist, auf das Vorhandensein einer MRI-Vorrichtung (3) zu schließen, wenn eine Vielzahl von Messwerten (S), die von der Erfassungsvorrichtung (103) erhalten werden, eine Zunahme einer Stärke des Magnetfeldes (M) anzeigt, **dadurch gekennzeichnet, dass** die Erfassungsvorrichtung (103) dazu ausgestaltet ist, Messungen mit einer spezifizierten Abtastrate zwischen 1 Hz und 50 Hz, insbesondere bei 4 Hz, durchzuführen und Messwerte (S) mit einer spezifizierten Abtastrate bereitzustellen, wobei die Verarbeitungsvorrichtung (101) dazu ausgestaltet ist, einen Zählerwert (C) zu erhöhen, wenn ein Messwert (S) eine Zunahme der Stärke des Magnetfelds (M) anzeigt, und den Zählerwert (C) auf einen Startwert zurückzusetzen, wenn der Zählerwert (C) in einer vordefinierten Zeitspanne nicht erhöht wird.

2. Implantierbare medizinische Vorrichtung (1) nach Anspruch 1, wobei die Verarbeitungsvorrichtung (101) dazu ausgestaltet ist, auf das Vorhandensein einer MRI-Vorrichtung (3) zu schließen, wenn eine Vielzahl von aufeinanderfolgenden Messwerten (S) eine Zunahme der Stärke des Magnetfeldes (M) anzeigen.

3. Implantierbare medizinische Vorrichtung (1) nach einem der vorhergehenden Ansprüche, wobei die Verarbeitungsvorrichtung (101) dazu ausgestaltet ist, den Zählerwert (C) zu erhöhen, wenn ein Messwert (S) eine Zunahme der Stärke des Magnetfeldes (M) anzeigt, die eine vordefinierte Zählschwelle (A1) überschreitet.

4. Implantierbare medizinische Vorrichtung (1) nach einem der vorhergehenden Ansprüche, wobei die Verarbeitungsvorrichtung (101) dazu ausgestaltet ist, den Zählerwert (C) zu vermindern, wenn ein Messwert (S) eine Abnahme der Stärke des Magnetfeldes (M) anzeigt, wobei die Abnahme der Stärke des Magnetfeldes eine vordefinierte Countdown-Schwelle überschreitet.

5. Implantierbare medizinische Vorrichtung (1) nach einem der vorhergehenden Ansprüche, wobei die Verarbeitungsvorrichtung (101) dazu ausgestaltet ist, auf das Vorhandensein einer MRI-Vorrichtung (3) zu schließen, wenn der Zählerwert (C) für eine vordefinierte Anzahl von Malen erhöht wird oder wenn der Zählerwert (C) eine vordefinierte Erkennungsschwelle (A3) erreicht oder überschreitet.

6. Implantierbare medizinische Vorrichtung (1) nach Anspruch 5, wobei die Verarbeitungsvorrichtung (101) dazu ausgestaltet ist, auf das Vorhandensein einer MRI-Vorrichtung (3) zu schließen, wenn der Zählerwert (C) für eine vordefinierte Anzahl von aufeinanderfolgenden Malen erhöht wird oder wenn der Zählerwert (C) eine vordefinierte Erkennungsschwelle (A3) erreicht oder überschreitet.

7. Implantierbare medizinische Vorrichtung (1) nach einem der vorhergehenden Ansprüche, wobei die Verarbeitungsvorrichtung (101) dazu ausgestaltet ist, den Zählerwert (C) auf einen Startwert zurückzusetzen, wenn ein Messwert (S) eine Abnahme der Stärke des Magnetfeldes (M) um einen Wert anzeigt, der größer ist als eine vordefinierte Rücksetzschwelle (A2).

8. Implantierbare medizinische Vorrichtung (1) nach einem der vorhergehenden Ansprüche, wobei die Verarbeitungsvorrichtung (101) dazu ausgestaltet ist, den Zählerwert (C) auf einen Startwert zurückzusetzen, wenn eine vordefinierte Anzahl von aufeinanderfolgenden Messwerten (S) jeweils eine Abnahme der Stärke des Magnetfeldes (M) um einen Wert anzeigen, der größer als eine vordefinierte Countdown-Schwelle ist.

9. Implantierbare medizinische Vorrichtung (1) nach einem der vorhergehenden Ansprüche, wobei die Verarbeitungsvorrichtung (101) dazu ausgestaltet ist, den Betrieb der implantierbaren medizinischen Vorrichtung (1) zu modifizieren, wenn darauf geschlossen wurde, dass eine MRI-Vorrichtung (3) vorhanden ist.

10. Implantierbare medizinische Vorrichtung (1) nach Anspruch 9, der sich auf einen der Ansprüche 4 bis 7 bezieht, wobei die Verarbeitungsvorrichtung (101) dazu ausgestaltet ist
- die Modifizierung des Betriebs der implantierbaren medizinischen Vorrichtung (1) zu beenden, wenn der Zählerwert (C) zurückgesetzt wird, oder
- einen Zeitgeber für die Beendigung zu starten, wenn der Zählerwert (C) zurückgesetzt wird, und die Modifizierung des Betriebs der implantierbaren medizinischen Vorrichtung (1) zu beenden, wenn der Zeitgeber für die Beendigung eine vordefinierte Zeitspanne erreicht hat.

11. Implantierbare medizinische Vorrichtung (1) nach einem der vorhergehenden Ansprüche, wobei die Verarbeitungsvorrichtung (101) dazu ausgestaltet ist, auf das Vorhandensein einer MRI-Vorrichtung (3) zu schließen, wenn zumindest ein Teil einer Kurve, die der Vielzahl von aufeinanderfolgenden Messwerten (S) zugeordnet ist, mit zumindest einem Teil einer Referenzkurve übereinstimmt.

12. Verfahren zum Betreiben einer implantierbaren medizinischen Vorrichtung (1), umfassend: Erfassen eines Magnetfeldes (M) mittels einer Erfassungsvorrichtung (103) der implantierbaren medizinischen Vorrichtung (1); und Erkennen eines Vorhandenseins einer MRI-Vorrichtung (3) mittels einer Verarbeitungsvorrichtung (101) der implantierbaren medizinischen Vorrichtung (1) auf der Grundlage von Messwerten (S), die von der Erfassungsvorrichtung (103) erhalten werden; Schließen, durch die Verarbeitungsvorrichtung (101), darauf, dass eine MRI-Vorrichtung (3) vorhanden ist, wenn eine Vielzahl von Messwerten (S), die von der Erfassungsvorrichtung (103) erhalten werden, eine Zunahme einer Stärke des Magnetfelds (M) anzeigt, **gekennzeichnet durch** Durchführen von Messungen mit einer spezifizierten Abtastrate zwischen 1 Hz und 50 Hz, insbesondere bei 4 Hz, und Bereitstellen von Messwerten (S) mit der spezifizierten Abtastrate, Erhöhen eines Zählerwerts (C) durch die Verarbeitungsvorrichtung (101), wenn ein Messwert (S) eine Zunahme der Stärke des Magnetfelds (M) anzeigt, und Zurücksetzen des Zählerwerts (C) auf einen Startwert durch die Verarbeitungsvorrichtung (101), wenn der Zählerwert (C) in einer vordefinierten Zeitspanne nicht erhöht wird.

## Revendications

1. Dispositif médical implantable (1), comprenant : un dispositif de détection (103) pour détecter un champ magnétique (M) ; et un dispositif de traitement (101) conçu pour détecter une présence d'un dispositif d'IRM (3) en se basant sur les valeurs de mesure (S) obtenues auprès du dispositif de détection (103) ; dans lequel le dispositif de traitement (101) est conçu pour conclure qu'un dispositif d'IRM (3) est présent si une multiplicité de valeurs de mesure (S) obtenues auprès du dispositif de mesure (103) indique une augmentation d'une intensité du champ magnétique (M), **caractérisé en ce que** le dispositif de détection (103) est conçu pour réaliser des mesures à une fréquence d'échantillonnage spécifiée entre 1 Hz et 50 Hz, en particulier à 4 Hz, et pour fournir des valeurs de mesure (S) à la fréquence d'échantillonnage spécifiée,
dans lequel le dispositif de traitement (101) est conçu pour incrémenter une valeur de compteur (C) si une valeur de mesure (S) indique une augmentation de l'intensité du champ magnétique (M) et pour réinitialiser la valeur de compteur (C) jusqu'à une valeur de départ si la valeur de compteur (C) n'est pas augmentée dans une période prédéfinie.

2. Dispositif médical implantable (1) selon la revendication 1, dans lequel le dispositif de traitement (101) est conçu pour conclure qu'un dispositif d'IRM (3) est présent si une multiplicité de valeurs de mesure consécutives (S) indique une augmentation de l'intensité du champ magnétique (M).

3. Dispositif médical implantable (1) selon l'une des revendications précédentes, dans lequel le dispositif de traitement (101) est conçu pour incrémenter la valeur de compteur (C) si une valeur de mesure (S) indique une augmentation de l'intensité du champ magnétique (M) dépassant un seuil de décompte prédéfini (A1).

4. Dispositif médical implantable (1) selon l'une des revendications précédentes, dans lequel le dispositif de traitement (101) est conçu pour décrémenter la valeur de compteur (C) si une valeur de mesure (S) indique une diminution de l'intensité du champ magnétique (M), dans lequel la diminution de l'intensité du champ magnétique dépasse un seuil de compte à rebours prédéfini.

5. Dispositif médical implantable (1) selon l'une des revendications précédentes, dans lequel le dispositif de traitement (101) est conçu pour conclure qu'un dispositif d'IRM (3) est présent si la valeur de compteur (C) est augmentée un nombre prédéfini de fois ou si la valeur de compteur (C) atteint ou dépasse un seuil de détection prédéfini (A3).

6. Dispositif médical implantable (1) selon la revendication 5, dans lequel le dispositif de traitement (101) est conçu pour conclure qu'un dispositif d'IRM (3) est présent si la valeur de compteur (C) est augmentée un nombre prédéfini de fois consécutives ou si la valeur de compteur (C) atteint ou dépasse un seuil de détection prédéfini (A3).

7. Dispositif médical implantable (1) selon l'une des revendications précédentes, dans lequel le dispositif de traitement (101) est conçu pour réinitialiser la valeur de compteur (C) à une valeur de départ si une valeur de mesure (S) indique une diminution de l'intensité du champ magnétique (M) d'une valeur supérieure à un seuil de réinitialisation prédéfini (A2).

8. Dispositif médical implantable (1) selon l'une des revendications précédentes, dans lequel le dispositif de traitement (101) est conçu pour réinitialiser la valeur de compteur (C) à une valeur de départ si un nombre prédéfini de valeurs de mesure (S) consécutives indiquent chacune une diminution de l'intensité du champ magnétique (M) d'une valeur supérieure à un seuil de compte à rebours prédéfini.

9. Dispositif médical implantable (1) selon l'une des revendications précédentes, dans lequel le dispositif de traitement (101) est conçu pour modifier le fonctionnement du dispositif médical implantable (1) s'il est conclu qu'un dispositif d'IRM (3) est présent.

10. Dispositif médical implantable (1) selon la revendication 9 par référence à l'une des revendications 4 à 7, dans lequel le dispositif de traitement (101) est conçu pour
- achever la modification du fonctionnement du dispositif médical implantable (1) si la valeur de compteur (C) est réinitialisée, ou
- démarrer un chronomètre de fin si une valeur de compteur (C) est réinitialisée, et achever la modification du fonctionnement du dispositif médical implantable (1) lorsque le chronomètre de fin atteint une marge de temps prédéfinie.

11. Dispositif médical implantable (1) selon l'une des revendications précédentes, dans lequel le dispositif de traitement (101) est conçu pour conclure qu'un dispositif d'IRM (3) est présent si au moins une partie d'une courbe associée à la multiplicité de valeurs de mesure (S) consécutives correspond à au moins une partie d'une courbe de référence.

12. Méthode de fonctionnement d'un dispositif médical implantable (1), comprenant les étapes consistant à : détecter un champ magnétique (M) en utilisant un dispositif de détection (103) du dispositif médical implantable (1) ; et détecter une présence d'un dispositif d'IRM (3), en utilisant un dispositif de traitement (101) du dispositif médical implantable (1), en se basant sur les valeurs de mesure (S) obtenues auprès du dispositif de détection (103) ; conclure, au moyen du dispositif de traitement (101), qu'un dispositif d'IRM (3) est présent si une multiplicité de valeurs de mesure (S) obtenues auprès du dispositif de détection (103) indique une augmentation d'une intensité du champ magnétique (M), **caractérisée par** la réalisation de mesures à une fréquence d'échantillonnage spécifiée entre 1 Hz et 50 Hz, en particulier à 4 Hz, et fournir des valeurs de mesure (S) à la fréquence d'échantillonnage spécifiée, incrémenter, au moyen du dispositif de traitement (101), une valeur de compteur (C) si une valeur de mesure (S) indique une augmentation de l'intensité du champ magnétique (M) et réinitialiser, au moyen du dispositif de traitement (101), la valeur de compteur (C) jusqu'à une valeur de départ si la valeur de compteur (C) n'est pas augmentée dans une période prédéfinie.
